# EUROPEAN PATENT APPLICATION

(11) **EP 4 467 558 A1**
(43) Date of publication of application: **27.11.2024**
(21) Application number: 23740111.2
(22) Date of filing: 17.01.2023
(51) Int. Cl.: C07H 19/167, C07H 1/00, A61K 31/7076, A61P 31/18

(54) **CRYSTAL OF 4'-SUBSTITUTED NUCLEOSIDE, AND PREPARATION METHOD THEREFOR, COMPOSITION THEREOF AND USE THEREOF**

(30) Priority: 17.01.2022 CN 202210051832
(71) Applicant: Henan Genuine Biotech Co., Ltd., Henan 467036 (CN)
(72) Inventor: DU, Jinfa, Pingdingshan, Henan 467036 (CN)
(74) Representative: Bandpay & Greuter
(86) International application number: PCT/CN2023/072479
(87) International publication number: WO 2023/134770

(57) **Abstract**

Provided are a type XVIII crystal of compound (3), and a preparation method therefor, a composition containing the crystal, and the use thereof. The type XVIII crystal shows good physical properties, such as easily prepared and crystal form stability, which is convenient for large-scale production and quality control of drugs, and is suitable as a crystal form of compound (3) in drug preparation.

## Description

### TECHNICAL FIELD

The present disclosure relates to the field of pharmaceutical chemistry, specifically to a crystal of 4'-substituted nucleoside, and a preparation method therefor, a composition thereof and use thereof.

### BACKGROUND

HIV infection causes the onset of AIDS. Since the first case of AIDS was discovered in the United Stated in 1981, there have currently been approximately 39 million AIDS patients in the world. AIDS has become a maj or threat to human health. The replication of human immunodeficiency virus (HIV) is completed through processes such as adsorption, invasion and uncoating, reverse transcription, and synthesis, assembly, release and maturation of integrated viral RNA and proteins, etc. Each of these links could be used as a target for HIV-inhibiting drugs. After more than 30 years of research, 40 HIV therapeutic drugs including combined drugs have been approved by the U.S. FDA for clinical use. According to the mechanisms of action of these drugs, they can be mainly divided into several categories such as nucleoside and non-nucleoside reverse transcriptase inhibitors, protease inhibitors, invasion inhibitors, and integrase inhibitors. These drugs are effective in inhibiting the HIV replication, but none of them could cure AIDS. Moreover, after a period of treatment with these drugs, the drug resistance of viruses will continue to develop, making the existing drug therapies ineffective. Therefore, the combination of HIV inhibitors with different mechanisms of action has become a standard strategy for treatment of AIDS. Nevertheless, even if drugs are combined to treat AIDS, new drug resistance will emerge continuously. Thus, there still remains a need to continue to search for AIDS therapeutic drugs with new mechanisms of action. Additionally, to date, all AIDS therapeutic drugs have to be taken at least once a day, and long-term medication has become a burden for patients. Therefore, it is necessary to develop long-acting drugs for AIDS treatment.

2'-Deoxynucleosides are the main class of HIV inhibitors used in clinical practice, and they exert their drug effects by inhibiting reverse transcriptase. At present, all the clinical nucleoside inhibitors for HIV are 3'-deoxynucleosides. Owing to the absence of 3'-OH, the drug terminates the DNA elongation of HIV after embedded in the DNA of HIV, thereby becoming a terminator of DNA synthesis of HIV and thus achieving the effect of inhibiting the HIV replication. In recent years, nucleosides with 3'-OH, such as compound (I) (C.A. Stoddart et al Antimicrob. Agents Chemother. 2015, 59, 4190) and compound (II) (Q .Wang et al Eur. J. Med .Chem. 2011, 46, 4178) have been successively reported to have significant inhibitory activities against HIV Their structures are characterized by the presence of a large substituent at the 4'-position, such as the ethynyl group in compound (I) and the azide group in compound (2). As a result of introduction of these large substituents, the nucleoside phosphates are bound into the viral DNA, and then the elongation of viral DNA becomes slow or terminates due to the steric hindrance effect, thereby fulfilling the purpose of inhibiting the HIV replication.

Among adenine nucleosides, the introduction of 2-fluorine increases the HIV inhibitory activity (EC₅₀ = 11 nM for EdA) of 4'-ethynyl-2'-deoxyadenosine (EC₅₀ = 0.05 nM for Compound 1, EFdA) by 2200 times (E. Michailidis et al J. Biol. Chem. 2009, 284,35681).

The U.S. patent (J. Chang US8835615, 2014) discloses the chemical structure of compound (3) (2R,3R,4S,5R)-5-(6-amino-2-fluoro-9H-purin-9-yl)-2-ethynyl-4-fluoro-2-(hydroxymethyl)tetrahydrofuran-3-ol. Subsequently, the Chinese patent (Junbiao CHANG, CN109893536 B, 2021) discloses the activity against HIV (EC₅₀ = 0.9 nM) and safety (without significant cytotoxicity within the tested dose range, CC₅₀>8000 nM) of compound (3), and also discloses the crystal form A of compound (3). The DSC and TGA studies have found that the molecular weight of the compound is 311.24, and the weight loss of the crystal form A is 9.0% at 110°C, indicating that crystal form A contains a solvated crystal of one molecule of methanol (theoretical methanol content: 9.33%), and methanol will be lost upon heating.

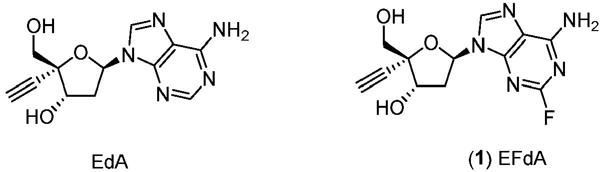

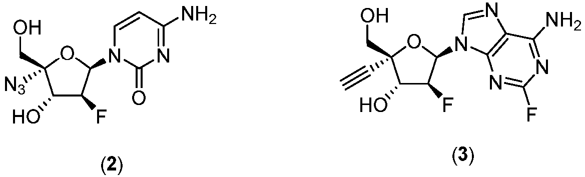

### Structures of Some Nucleoside Compounds under Research

### SUMMARY

The present inventors have conducted intensive studies on the crystal form of the compound (3) and found that compound (3) forms numerous crystal forms under different conditions. However, a majority of crystal forms are unstable. The present inventors have unexpectedly found the type XVIII crystal exhibits very excellent physical properties, such as ease of preparation and stable crystal form, which is convenient for mass production and quality control of drugs and is quite suitable as a crystal form of compound (3) in the preparation of a drug.

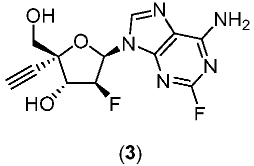

In view of the foregoing, the present disclosure provides a type XVIII crystal of a compound (3). The type XVIII crystal has characteristic peaks at diffraction angles of 20 values = 9.325°, 15.787°, and 16.55° in an X-ray powder diffraction pattern obtained by using Cu-Kα radiation, wherein the 20 values are allowed to vary within an error range.

The data on diffraction peaks of the type XVIII crystal of compound (3) are as shown in Table 1 below.

**Table 1. XRPD Data on Type XVIII Crystal**

| Pos. [°2θ] | Rel. Int. [%] |
|---|---|
| 9.325 | 27.9 |
| 15.787 | 14.4 |
| 16.55 | 31.6 |

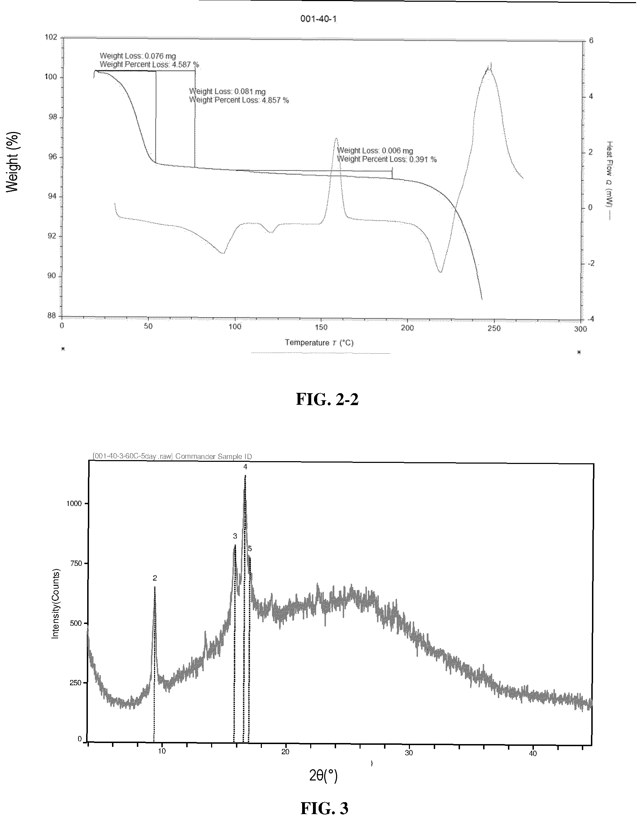

The XRPD pattern of the type XVIII crystal is as shown in FIG. 3.

In another aspect, the present disclosure provides a method for preparing a type XVIII crystal of compound (3), comprising: leaving a type XVI or XVII crystal at 40°C to 160°C, preferably 60°C to 90°C, more preferably 60°C for 1 to 10 days, preferably 5 to 10 days, more preferably 1 day to obtain the type XVIII crystal; preferably, the preparation step of the type XVI crystal comprises: suspending and stirring the compound (3) in an ethanol/water mixed solvent at a volume ratio of 10:1-90, preferably 10:1-50, more preferably 10:1-20 for 1 to 72 h, preferably, 1 to 50 h, more preferably 10 to 40 h, more preferably 24 h, and filtering to obtain the type XVI crystal; preferably, the type XVII crystal is obtained by heating the XVI crystal in a nitrogen atmosphere to 40°C to 58°C, preferably 55°C and holding for appropriate time; or obtained by vacuum drying the type XVI crystal at room temperature for 5 to 72 h, preferably 10 to 50 h, more preferably 24 h.

In another aspect, the present disclosure further provides a pharmaceutical composition, comprising the type XVIII crystal of compound (3) described above and an optional pharmaceutical excipient.

In another aspect, the present disclosure further provides use of a type XVIII crystal of a compound (3), or the above-mentioned pharmaceutical composition comprising the type XVIII crystal of the compound (3) and an optional pharmaceutical excipient in the preparation of a pharmaceutical or pharmaceutical formulation for resisting HIV or treating AIDS.

The present disclosure further provides a method for resisting HIV or treating AIDS, comprising administering, to a subject, an anti-HIV or therapeutically effective dose of a type XVIII crystal of a compound (3) or the above-mentioned pharmaceutical composition comprising the type XVIII crystal of the compound (3) and an optional pharmaceutical excipient.

### Advantageous Effects of the Invention

The present inventors have prepared a myriad of crystal forms of compound (3), most of which are unstable solid forms. The type XVIII crystal of compound (3) exhibits excellent physical properties, such as ease of preparation and stable crystal form, which facilitates mass production and quality control of drugs and suitable as the crystal form of compound (3) in the drug preparation.

### BRIEF DESCRIPTION OF THE DRAWINGS

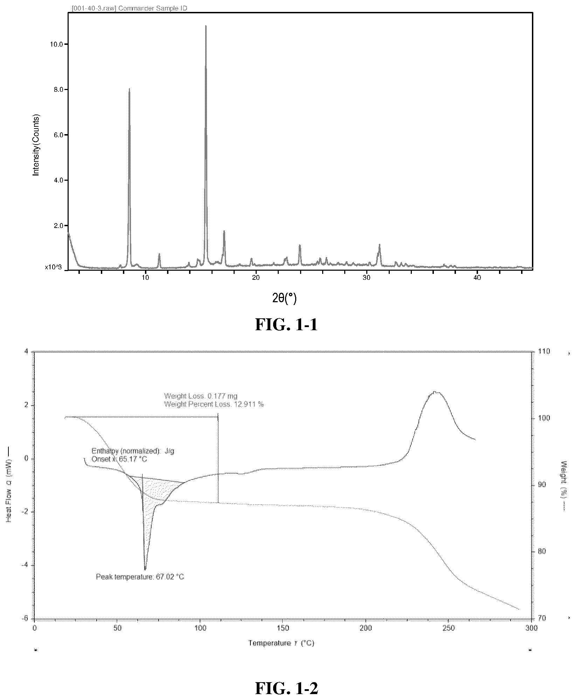

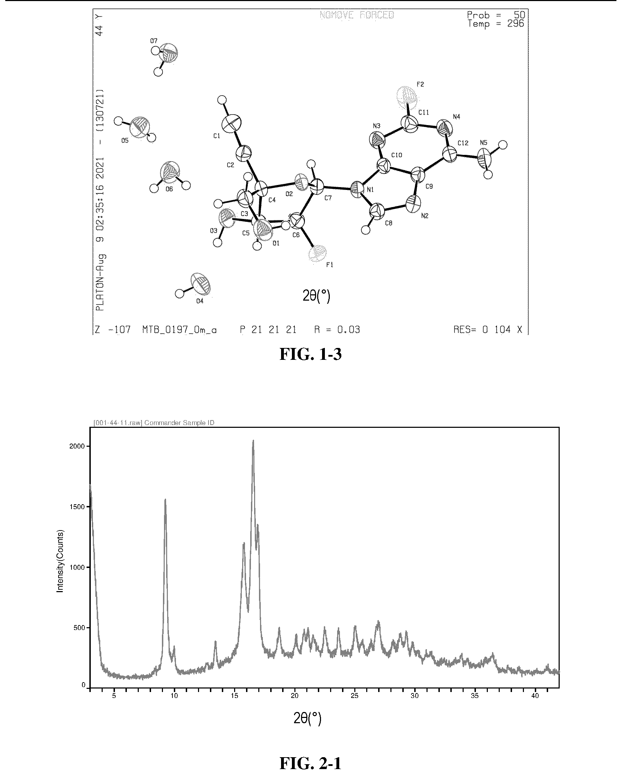

FIG. 1-1 shows the XRPD pattern of the type XVI crystal.
FIG. 1-2 shows the TGA/DSC diagram for type XVI crystal.
FIG. 1-3 shows the three-dimensional view of single-crystal X-ray diffraction molecules of the type XVI crystal.
FIG. 2-1 shows the XRPD pattern of the type XVII crystal.
FIG. 2-2 shows the DCS-TGA diagram for the type XVII crystal.
FIG. 3 shows the XRPD pattern of the type XVIII crystal.
FIG. 4 shows the XRPD pattern of the type I crystal.
FIG. 5 shows the TGA/DSC diagram for the type I crystal.
FIG. 6 shows the XRPD comparison diagram before and after the type I crystal is heated to 55°C.
FIG. 7 shows the XRPD pattern of the type I crystal when heated to 100°C.
FIG. 8 shows the XRPD pattern of the type II crystal.
FIG. 9 shows the XRPD pattern of the type IV crystal.
FIG. 10 shows the XRPD pattern of the type V crystal.
FIG. 11 shows the XRPD pattern of the type VI crystal.
FIG. 12 shows the XRPD pattern of the type VII crystal.
FIG. 13 shows the XRPD pattern of the type VIII crystal.
FIG. 14 shows the XRPD pattern of the type IX crystal.
FIG. 15 shows the XRPD pattern of the type X crystal.
FIG. 16 shows the XRPD pattern of the type XI crystal.
FIG. 17 shows the XRPD pattern of the type XII crystal.
FIG. 18 shows the XRPD pattern of the type XIII crystal.
FIG. 19 shows the XRPD comparison diagrams before and after coating and drying the type XIII crystal.
FIG. 20 shows the XRPD pattern of the type XIV crystal.
FIG. 21 shows the XRPD pattern of the type XV crystal.

### DETAILED DESCRIPTION

The present disclosure will be further illustrated below with reference to specific experiments. It should be appreciated that these experiments are merely intended to illustrate the present disclosure and are not intended to limit the scope of protection for the present disclosure. Experimental methods for which no specific conditions are indicated in the following experiments are generally carried out according to conventional conditions or the conditions recommended by the manufacturers. Unless otherwise defined, all professional and scientific terms used herein have the same meanings as typically understood by technicians in the art. In addition, any methods and materials similar or equivalent to those described herein may be applied to the methods of the present disclosure. The preferred implementation methods and materials as shown herein are for illustrative purposes only.

The starting materials used in the experiments of the present disclosure are known and are commercially available, or may be synthesized by the methods known in the art.

### Experiment 1

### Type XVI Crystal and Type XVII Crystal

Compound (3) was suspended and stirred for 24 h in an ethanol/water mixed solvent (at a volume ratio of 8:2) and filtered to obtain the type XVI crystal. The XRPD pattern was as shown in FIG. 1-1 and the DSC-TGA diagram was as shown in FIG. 1-2.

The single-crystal data on the type XVI crystal were as shown in Table 2.

**Table 2. Single-Crystal Data on Type XVI Crystal**

| | | |
|---|---|---|
| Labels | 001-40-1 (In Solvent) | |
| Test formula | C12 H19 F2 N5 O7 | |
| Molecular weight | 383.32 | |
| Temperature | 296(2) K | |
| Wavelength | 1.54178 Å | |
| Crystal system | Orthorhombic | |
| Space group | P2₁2₁2₁ | |
| Cell parameters | a = 6.7413(7) Å | a= 90°. |
| | b = 11.9385(13) Å | b= 90°. |
| | c = 20.906(2) Å | g = 90°. |
| Volume | 16825(3) Å³ | |
| Z | 4 | |
| Density (calculated value) | 1.513 Mg/m³ | |

The three-dimensional molecular view of the single-crystal X-ray diffraction of the type XVI crystal was as shown in FIG. 1-3, indicating that each type XVI crystal contained four waters of crystallization.

After vacuum drying at room temperature for 24 h, the type XVI crystal showed an obvious dehydration phenomenon (weight loss: 14.39%), and transformed into a monohydrate crystal form - type XVII crystal after losing three waters of crystallization. The XRPD pattern was as shown in FIG. 2-1 and the DCS-TGA diagram was as shown in FIG. 2-2.

The type XVI crystal was heated to 55°C in a nitrogen atmosphere and held for 2 to 24 h. The XRPD pattern showed that the type XVII crystal was also obtained.

The type XVII crystal was dried at 90°C for 1 to 24 h and lost one molecule of water (weight loss: 5.47%) to obtain the type XVIII crystal.

### Experiment 2

### Type XVIII Crystal

The type XVI crystal was left at 60°C for 5 days to obtain a type XVIII crystal, which was an anhydrous crystal. The LC-MS detection and analysis showed that the crystal did not undergo chemical changes.

The type XVIII crystal had characteristic peaks at diffraction angles of 2θ values = 9.325°, 15.787°, and 16.55° in the X-ray powder diffraction pattern obtained by using the Cu-Kα radiation, where the 20 values were allowed to vary within the error range.

The data on diffraction peaks of the type XVIII crystal of compound (3) were as shown in Table 1 above.

The XRPD pattern of the type XVIII crystal was as shown in FIG. 3.

### Experiment 3

### Stability of Type XVI Crystal, Type XVII Crystal and Type XVIII Crystal

The type XVI crystal and the type XVII crystal were left at 60±5°C for 5 days, respectively. The XRPD pattern showed that both of them were transformed into the type XVIII crystals.

The type XVII crystal was left for 5 days under the conditions of 25°C and 92.5±5% humidity. The XRPD pattern showed that the type XVI crystal was obtained.

The type XVIII crystal was heated to 150°C or left for 24 h under the conditions of 25°C and 92.5±5% humidity. The XRPD pattern showed that the crystal form did not change.

### Experiment 4

### Type I Crystal

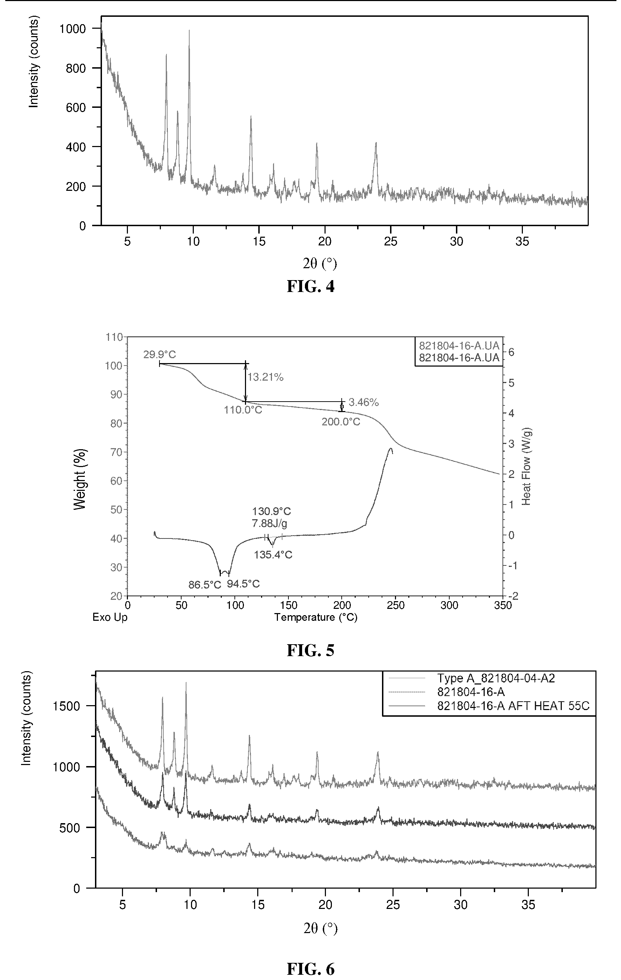

The solid of compound (3) was dissolved in 2-MeTHF, and n-heptane was added dropwise at room temperature and stirred for 4 h to precipitate a crystal. The crystal was filtered and vacuum dried to obtain the type I crystal of compound (3). The XRPD pattern was as shown in FIG. 4.

The type I crystal of compound (3) had characteristic peaks at diffraction angles of 2θ values = 7.94°, 8.79°, 9.68°, 11.56°, 13.74°, 14.37°, 15.98°, 17.78°, 19.39°, and 23.85° in an X-ray powder diffraction pattern obtained by using Cu-Kα radiation.

**Table 3. XRPD Data on Type I Crystal**

| Peak Position [°2θ] | Relative Peak Intensity [%] |
|---|---|
| 7.94 | 79.23 |
| 8.79 | 44.33 |
| 9.68 | 100.00 |
| 11.56 | 11.92 |
| 13.74 | 11.45 |
| 14.37 | 51.34 |
| 15.98 | 12.22 |
| 17.78 | 4.68 |
| 19.39 | 33.41 |
| 23.85 | 35.60 |

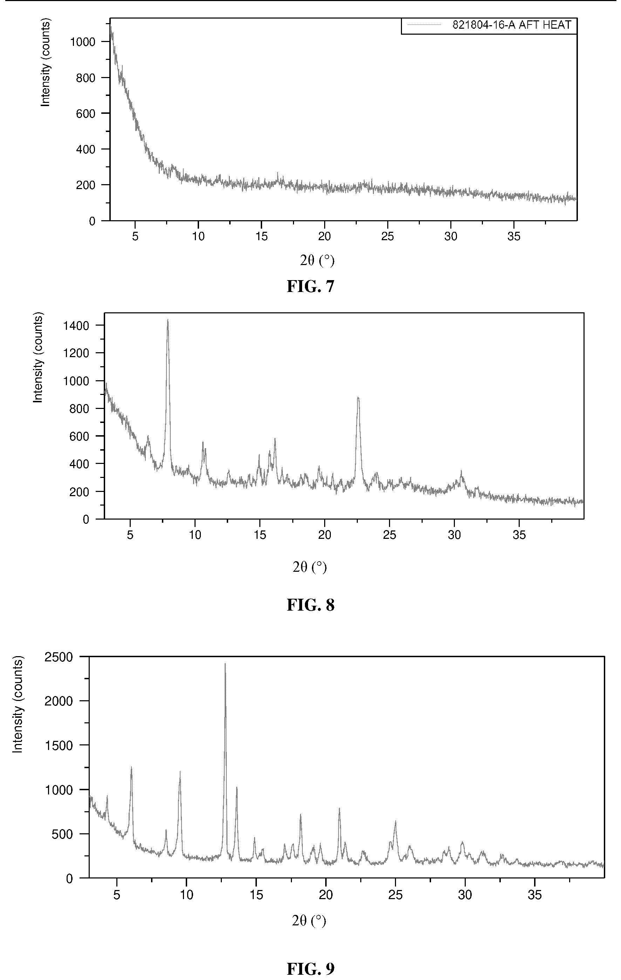

The TGA/DSC diagram for the type I crystal of compound (3) was as shown in FIG. 5. The crystal suffered from a weight loss of 13.2% when heated to 110°C, and a weight loss of 3.5% when further heated to 200°C. Three endothermic signals occurred in the range of 80°C to 140°C. The type I crystal was heated to 55°C under nitrogen protection and then cooled to room temperature. The sample was removed and exposed to air to test XRPD. The results were as shown in FIG. 6, where the upper curve was the original diagram for the crystal form I, the middle curve was the XRPD pattern before heating, and the lower curve was the XRPD pattern after heating to 55°C, showing that the diffraction peak intensity of the sample was somewhat decreased. The NMR results showed that the molar ratio of 2-MeTHF to API was 0.25:1.00 (6.5 wt%) after heating, and 2-MeTHF was reduced by about 3.5 wt% compared to that before heating. After the type I crystal was heated to 100°C under the same conditions of nitrogen protection, the XRPD results were as shown in FIG. 7, showing that the crystal had been transformed into an amorphous crystal. The NMR results showed that the molar ratio of 2-MeTHF to API was 0.09:1.00 (2.4 wt%) after heating, and 2-MeTHF was reduced by about 7.6 wt% compared to that before heating.

### Experiment 5

### Type II Crystal

The solid of compound (3) was dissolved into 1,4-dioxane/DCM (1-5/10), and n-heptane was added dropwise to the solution at room temperature and stirred for 4 h to precipitate a crystal. The crystal was filtered and vacuum dried to obtain the type II crystal of compound (3). The XRPD pattern was as shown in FIG. 8.

The type II crystal had characteristic peaks at diffraction angles of 20 values = 6.39°, 7.84°, 10.58°, 12.57°, 14.88°, 15.71°, 16.12°, 19.6°, 22.57°, 23.91°, and 30.62° in an X-ray powder diffraction pattern obtained by using Cu-Kα radiation.

**Table 4. XRPD Data on Type II Crystal**

| Peak Position [°2θ] | Relative Peak Intensity [%] |
|---|---|
| 6.39 | 12.49 |
| 7.84 | 100.00 |
| 10.58 | 26.61 |
| 12.57 | 8.65 |
| 14.88 | 15.36 |
| 15.71 | 22.47 |
| 16.12 | 31.30 |
| 19.60 | 8.47 |
| 22.57 | 62.65 |
| 23.91 | 6.93 |
| 30.62 | 10.91 |

The type II crystal changed in the crystal form after vacuum drying at room temperature for half an hour and transformed into a type III crystal. As the type II crystal was unstable and had been transformed after dying for a short period of time, no further studies were carried out on the type II crystal.

### Experiment 6

### Type IV Crystal

The solid of compound (3) was suspended and stirred in EtOH for 48 h at room temperature, and filtered to obtain the type IV crystal. The XRPD pattern was as shown in FIG. 9.

The type IV crystal had characteristic peaks at diffraction angles of 20 values = 4.28°, 6.03°, 8.50°, 9.53°, 12.76°, 13.58°, 14.84°, 15.45°, 17.02°, 17.61°, 18.17°, 19.12°, 19.60°, 20.97°, 21.38°, 22.66°, 24.99°, 25.99°, 28.47°, 28.82°, 29.80°, 31.24°, 32.66°, and 36.78° in an X-ray powder diffraction pattern obtained by using Cu-Kα radiation.

**Table 5. XRPD Data on Type IV Crystal**

| Peak Position [°2θ] | Relative Peak Intensity [%] |
|---|---|
| 4.28 | 11.98 |
| 6.03 | 38.66 |
| 8.50 | 11.78 |
| 9.53 | 41.18 |
| 12.76 | 100.00 |
| 13.58 | 37.85 |
| 14.84 | 10.63 |
| 15.45 | 6.39 |
| 17.02 | 8.33 |
| 17.61 | 9.35 |
| 18.17 | 24.53 |
| 19.12 | 7.80 |
| 19.60 | 9.64 |
| 20.97 | 28.26 |
| 21.38 | 11.19 |
| 22.66 | 604 |
| 24.99 | 21.87 |
| 25.99 | 904 |
| 28.47 | 6.38 |
| 28.82 | 8.04 |
| 29.80 | 11.07 |
| 31.24 | 5.98 |
| 32.66 | 4.22 |
| 36.78 | 1.46 |

After the type IV crystal was vacuum dried at room temperature for 15 min, decreased relative intensity of the diffraction peaks could be observed. After the dried crystal was left closed at room temperature for about 5 days, increased relative intensity of the diffraction peaks and increased number of diffraction peaks could be observed. The analysis showed that the type IV crystal might undergo solvent or water removal during vacuum drying, and adsorb the moisture in the air upon contact with air, thereby increasing the degree of crystallinity.

TGA showed that the type IV crystal was unstable at 120°C with the weight loss of 10.6%, which was a monohydrate crystal form. Under the conditions of nitrogen protection, the type IV crystal was heated to 141°C and then tested for XRPD. The results showed that the type IV crystal was transformed into a stable type III crystal at 141°C.

### Experiment 7

### Type V Crystal

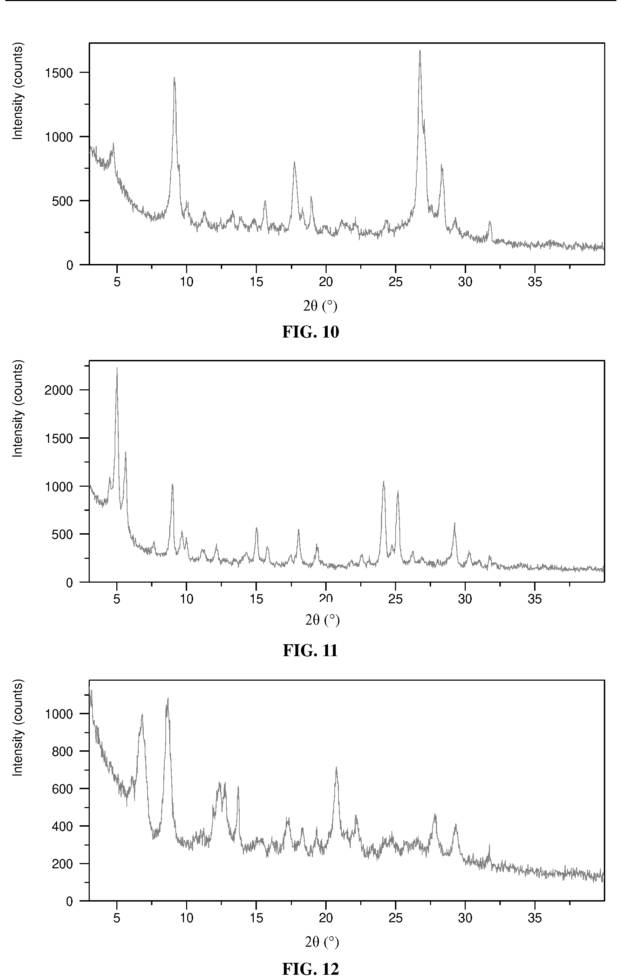

The solid of compound (3) was suspended and stirred in EtOAc for 48 h at room temperature, filtered, and vacuum dried at room temperature for 15 min to obtain the type V crystal. The XRPD pattern was as shown in FIG. 10.

The type V crystal had characteristic peaks at diffraction angles of 20 values = 4.73°, 9.12°, 11.27°, 13.22°, 15.58°, 17.69°, 18.95°, 21.13°, 22.10°, 24.29°, 26.72°, 27.09°, 28.34°, 29.25°, and 31.76° in an X-ray powder diffraction pattern obtained by using Cu-Kα radiation.

**Table 6. XRPD Data on Type V Crystal**

| Peak Position [°2θ] | Relative Peak Intensity [%] |
|---|---|
| 4.73 | 18.22 |
| 9.12 | 73.14 |
| 11.27 | 6.49 |
| 13.22 | 6.45 |
| 15.58 | 14.84 |
| 17.69 | 36.96 |
| 18.95 | 17.63 |
| 21.13 | 6.37 |
| 22.10 | 6.14 |
| 24.29 | 8.50 |
| 26.72 | 100.00 |
| 27.09 | 54.66 |
| 28.34 | 37.44 |
| 29.25 | 10.32 |
| 31.76 | 10.93 |

The TGA/DSC test results showed that the type V crystal suffered from a weight loss of 15.4% when heated to 140°C, and a weight loss of 2.8% when further heated to 240°C. Multiple thermal signals could be observed in the range of 77°C to 203°C. The NMR results showed that the molar ratio of EtOAc to API was 0.40:1.00 (10.2 wt%).

The type V crystal was subjected to the variable-temperature XRPD test. A significant decrease in degree of crystallinity was observed after the sample was purged with nitrogen for about 20 min. The type V crystal was substantially transformed into an amorphous crystal after heating to 90°C and 130°C under the nitrogen protection, and transformed into another crystal after further heated to 154°C. Analyses of the NMR and TGA results showed that the type V crystal was a solvate.

### Experiment 8

### Type VI Crystal

The solid of compound (3) was suspended and stirred in MIBK (methyl isobutyl ketone) at room temperature for 48 h, filtered, and vacuum dried at room temperature for 15 min to obtain the type VI crystal. The XRPD was as shown in FIG. 11.

The type VI crystal had characteristic peaks at diffraction angles of 20 values = 5.01°, 5.62°, 7.62°, 8.98°, 9.67°, 9.99°, 11.11°, 12.12°, 14.22°, 15.02°, 15.79°, 18.01°, 19.37°, 22.55°, 24.18°, 25.15°, 26.22°, 29.21°, 30.29°, 31.74°, and 34.00° in an X-ray powder diffraction pattern obtained by using Cu-Kα radiation.

**Table 7. XRPD Data on Type VI Crystal**

| Peak Position [°2θ] | Relative Peak Intensity [%] |
|---|---|
| 5.01 | 100.00 |
| 5.61 | 52.70 |
| 7.62 | 6.47 |
| 8.98 | 47.48 |
| 9.67 | 15.82 |
| 9.99 | 11.86 |
| 11.11 | 6.94 |
| 12.12 | 950 |
| 14.22 | 5.81 |
| 15.02 | 22.06 |
| 15.79 | 10.91 |
| 18.01 | 21.26 |
| 19.37 | 11.80 |
| 22.55 | 7.32 |
| 24.18 | 52.18 |
| 25.15 | 48.50 |
| 26.22 | 8.15 |
| 29.21 | 27.36 |
| 30.29 | 8.21 |
| 31.74 | 6.34 |
| 34.00 | 1.75 |

The TGA/DSC results showed that the type VI crystal suffered from a weight loss of 4.9% when heated to 70°C, and a weight loss of 5.9% when further heated to 130°C. Multiple thermal signals could be observed in the range of 98°C to 210°C.

The type VI crystal was heated to 160°C under the nitrogen protection. The XRPD results showed that the crystal form was transformed.

### Experiment 9

### Type VII Crystal

At room temperature, the solid of compound (3) was suspended and stirred for 48 h in an IPA/toluene (v/v, 1:1) mixed solvent, filtered, and vacuum dried at room temperature for 15 min to obtain the type VII crystal. The XRPD results were as shown in FIG. 12.

The type VII crystal had characteristic peaks at diffraction angles of 20 values = 6.80°, 8.59°, 12.36°, 12.77°, 13.68°, 17.24°, 18.30, 19.32°, 20.77°, 22.20°, 24.47°, 27.81°, 29.32°, and 31.67° in an X-ray powder diffraction pattern obtained by using Cu-Kα radiation.

**Table 8. XRPD Data on Type VII Crystal**

| Peak Position [°2θ] | Relative Peak Intensity [%] |
|---|---|
| 6.80 | 76.64 |
| 8.59 | 100.00 |
| 12.36 | 45.49 |
| 12.77 | 38.03 |
| 13.68 | 47.03 |
| 17.24 | 22.07 |
| 18.30 | 17.43 |
| 19.32 | 13.81 |
| 20.77 | 63.08 |
| 22.20 | 25.93 |
| 24.47 | 6.55 |
| 27.81 | 27.02 |
| 29.32 | 23.87 |
| 31.67 | 8.90 |

The TGA/DSC test results showed that the type VII crystal suffered from a weight loss of 11.2% when heated to 130°C, and multiple thermal signals could be observed within the range of 88°C to 215°C. Under the nitrogen protection, the type VII crystal was heated to 130°C and then cooled to room temperature. The sample was removed and exposed to the air to test XRPD. The results showed that the crystal form was transformed.

### Experiment 10

### Type VIII Crystal

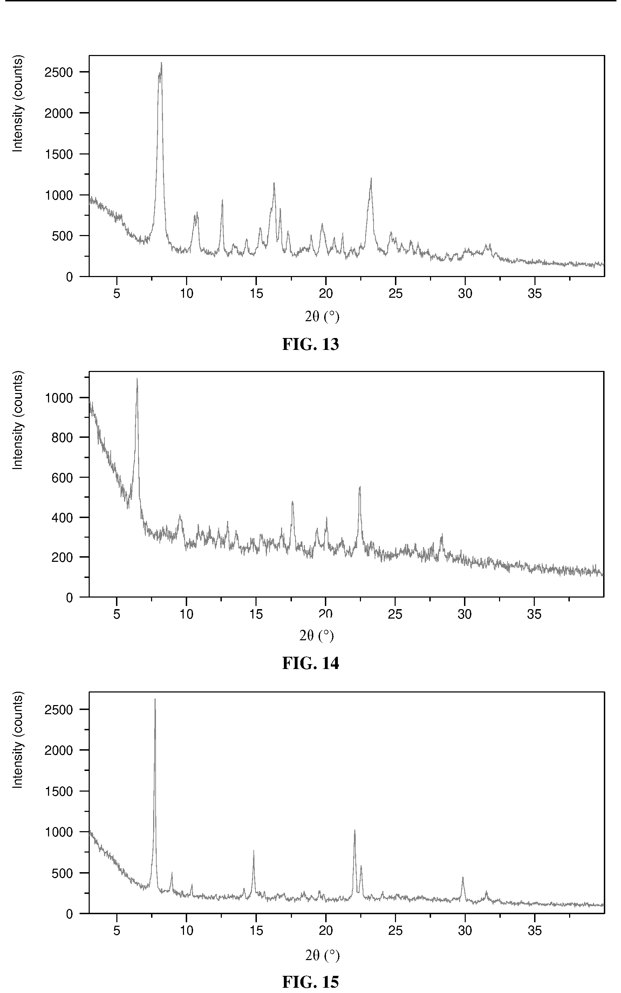

The solid of compound (3) was suspended and stirred in a THF/n-heptane (v/v, 1:1) mixed solvent at 50°C for 48 h, filtered, and vacuum dried at room temperature for 15 min to obtain the type VIII crystal. The XRPD pattern was as shown in FIG. 13.

The **type VIII crystal** had characteristic peaks at diffraction angles of 2θ values = 5.24°, 7.98°, 8.20°, 10.51°, 10.77°, 12.55°13.40°, 14.27°, 15.28°, 16.29°, 16.69°, 17.25°, 18.94°, 19.72°, 21.19°, 23.23°, 24.63°, 26.09°, 26.60°, 30.05°, and 31.77° in an X-ray powder diffraction pattern obtained by using Cu-Kα radiation.

**Table 9. XRPD Diffraction Peak Data on Type VIII Crystal Form**

| Peak Position [°2θ] | Relative Peak Intensity [%] |
|---|---|
| 5.24 | 4.06 |
| 7.98 | 95.96 |
| 8.20 | 100.00 |
| 10.51 | 16.80 |
| 10.77 | 19.75 |
| 12.55 | 2904 |
| 13.40 | 4.75 |
| 14.27 | 8.18 |
| 15.28 | 15.07 |
| 16.29 | 38.51 |
| 16.69 | 25.75 |
| 17.25 | 13.01 |
| 18.94 | 10.57 |
| 19.72 | 17.57 |
| 21.19 | 11.64 |
| 23.23 | 43.54 |
| 24.63 | 13.72 |
| 26.09 | 908 |
| 26.60 | 8.13 |
| 30.05 | 3.73 |
| 31.77 | 7.96 |

The TGA/DSC results showed that the type VIII crystal suffered from a weight loss of 10.6% when heated to 160°C, and multiple thermal signals could be observed within the range of 120°C to 220°C. Under the nitrogen protection, the type VIII crystal was heated to 160°C and then cooled to room temperature. The sample was removed and exposed to the air to test XRPD. The results showed that the crystal form had been transformed.

### Experiment 11

### Type IX Crystal

At room temperature, the compound (3) was dissolved in a THF/CHCl₃ mixed solvent and volatilized at room temperature to obtain the type IX crystal. The XRPD pattern was as shown in FIG. 14.

The type IX crystal had characteristic peaks at diffraction angles of 20 values = 6.47°, 9.57°, 13.56°, 16.84°, 17.61°, 19.36°, 20.06°, 22.45°, and 28.32° in an X-ray powder diffraction pattern obtained by using Cu-Kα radiation.

**Table 10. XRPD Data on Type IX Crystal**

| Peak Position [°2θ] | Relative Peak Intensity [%] |
|---|---|
| 6.47 | 100.00 |
| 9.57 | 17.93 |
| 13.56 | 8.20 |
| 16.84 | 9.22 |
| 17.61 | 35.46 |
| 19.36 | 15.63 |
| 2006 | 21.60 |
| 22.45 | 49.26 |
| 28.32 | 13.96 |

The TGA/DSC results showed that the type IX crystal suffered from a weight loss of 20.3% when heated to 160°C, and multiple thermal signals could be observed within the range of 130°C to 215°C. After the type IX crystal was left closed at room temperature for about 3 days, a reduced degree of crystallinity could be observed. The crystal was heated to 95°C under nitrogen protection and then cooled to room temperature. The sample was removed and exposed to the air to test XRPD. The results showed that the degree of crystallinity was further decreased. The crystal was heated to 160°C under the same conditions of nitrogen protection and then cooled to room temperature. The sample was removed and exposed to the air to test XRPD. The results showed that its crystal form had been transformed. The analyses of the NMR and TGA results showed that after heating, the change in crystal form was caused by solvent removal, and the type IX crystal was a solvate.

### Experiment 12

### Type X Crystal

The solid of compound (3) was suspended and stirred in IPAc (isopropyl acetate) at 50°C for 48 h, and filtered to obtain the type X crystal. The XRPD pattern was as shown in FIG. 15.

The type X crystal had characteristic peaks at diffraction angles of 20 values = 7.72°, 8.91°, 14.10°, 14.79°, 16.95°, 18.39°, 19.53°, 22.06°, 22.53°, 29.81°, and 31.51° in an X-ray powder diffraction pattern obtained by using Cu-Kα radiation.

**Table 11. XRPD Data on Type X Crystal**

| Peak Position [°2θ] | Relative Peak Intensity [%] |
|---|---|
| 7.72 | 100.00 |
| 8.91 | 8.59 |
| 14.10 | 4.77 |
| 14.79 | 24.51 |
| 16.95 | 2.57 |
| 18.39 | 2.79 |
| 19.53 | 4.00 |
| 22.06 | 35.63 |
| 22.53 | 17.81 |
| 29.81 | 12.77 |
| 31.51 | 4.98 |

The TGA/DSC results showed that the type X crystal suffered from a weight loss of 7.9% when heated to 130°C, and a weight loss of 4.5% when further heated to 160°C. Multiple thermal signals could be observed within the range of 126°C to 210°C.

The type X crystal was subjected to the variable-temperature XRPD test. The results showed that the position of the diffraction peak shifted after the X crystal was heated to 120°C while purging with nitrogen; the diffraction peak intensity was significantly reduced after the type X crystal was further heated to 130°C; and the crystal form was transformed after the type X crystal was further heated to 145°C.

### Experiment 13

### Type XI Crystal

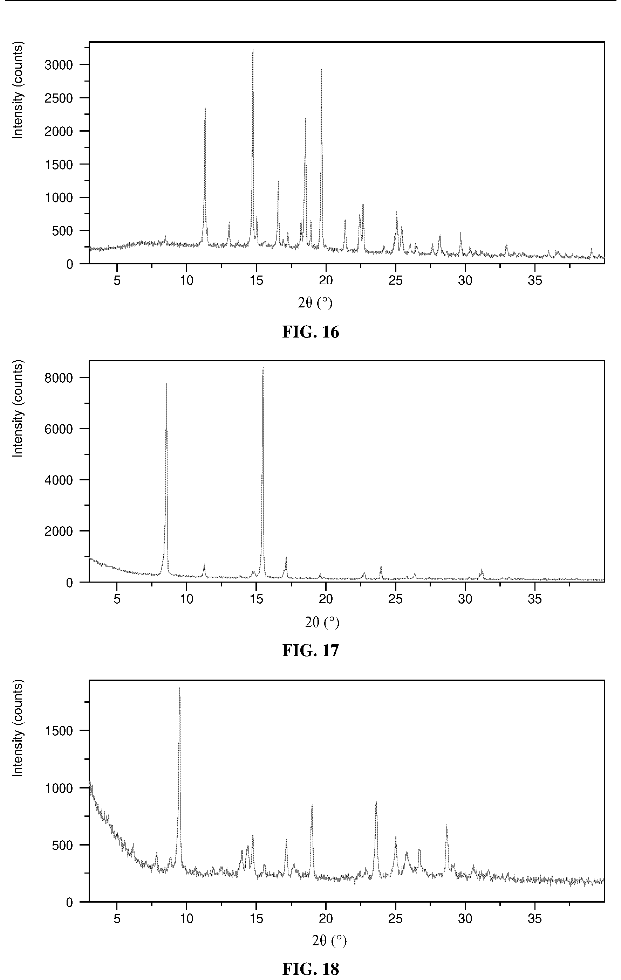

The solid of compound (3) was suspended and stirred in methyl tert-butyl ether for 48 h and filtered to obtain the type XI crystal. The XRPD pattern was as shown in FIG. 16.

The type XI crystal had characteristic peaks at diffraction angles of 20 values = 8.42°, 11.29°, 13.02°, 14.72°, 15.01°, 16.55°, 17.23°, 18.49°, 18.90°, 19.67°, 21.37°, 22.42°, 22.67°, 24.14°, 25.08°, 25.44°, 26.02°, 26.44°, 27.63°, 28.14°, 29.67°, 30.32°, 32.95°, 35.96°, 36.59°, and 39.03° in an X-ray powder diffraction pattern obtained by using Cu-Kα radiation, wherein the error range of the 20 values was ±0.2.

**Table 11. XRPD Data on Type XI Crystal**

| Peak Position [°2θ] | Relative Peak Intensity [%] |
|---|---|
| 8.42 | 2.71 |
| 11.29 | 69.01 |
| 13.02 | 12.26 |
| 14.72 | 100.00 |
| 15.01 | 15.81 |
| 16.55 | 33.47 |
| 17.23 | 8.14 |
| 18.49 | 64.94 |
| 18.90 | 15.41 |
| 19.67 | 89.95 |
| 21.37 | 17.08 |
| 22.42 | 19.58 |
| 22.67 | 24.34 |
| 24.14 | 3.09 |
| 25.08 | 20.61 |
| 25.44 | 13.66 |
| 26.02 | 5.29 |
| 26.44 | 4.10 |
| 27.63 | 5.32 |
| 28.14 | 8.51 |
| 29.67 | 9.51 |
| 30.32 | 4.48 |
| 32.95 | 6.76 |
| 35.96 | 2.97 |
| 36.59 | 2.28 |
| 39.03 | 3.97 |

The TGA/DSC test results showed that the weight loss of the XI crystal was 19.3% when heated to 120°C, and multiple thermal signals occurred at approximate 95°C to 210°C.

### Experiment 14

### Type XII Crystal

The type III crystal or type XI crystal of compound (3) was suspended and stirred in purified water at room temperature for 24 h, filtered, and open dried overnight at room temperature to obtain the type XII crystal. The XRPD pattern was as shown in FIG. 17.

The type XII crystal had characteristic peaks at diffraction angles of 20 values = 8.52°, 11.25°, 14.78°, 15.45°, 17.10°, 19.58°, 22.74°, 23.94°, 26.36°, 30.28°, 31.17°, and 33.11° in an X-ray powder diffraction pattern obtained by using Cu-Kα radiation.

**Table 13. XRPD Data on Type XII Crystal**

| Peak Position [°2θ] | Relative Peak Intensity [%] |
|---|---|
| 8.52 | 80.60 |
| 11.25 | 6.48 |
| 14.78 | 2.11 |
| 15.45 | 100.00 |
| 17.10 | 8.59 |
| 19.58 | 2.16 |
| 22.74 | 2.54 |
| 23.94 | 6.26 |
| 26.36 | 2.69 |
| 30.28 | 0.89 |
| 31.17 | 4.74 |
| 33.11 | 0.99 |

The type XII crystal was subjected to vacuum drying at room temperature for 10 min and transformed into an amorphous crystal. The samples open dried at room temperature were characterized by TGA/DSC. The results showed that the weight loss was 59.6% when the samples were heated to 120°C, and multiple thermal signals occurred within the range of 50°C to 240°C.

### Experiment 15

### Type XIII Crystal

The type III crystal or type XII crystal was suspended and stirred in methanol at room temperature for about 24 h, and filtered to obtain the type XIII crystal. The XRPD pattern was as shown in FIG. 18.

The type XIII crystal had characteristic peaks at diffraction angles of 2θ values = 6.15°, 7.81°, 9.46°, 13.92°, 14.39°, 14.72°, 15.56°, 17.13°, 17.71°, 18.99°, 23.59°, 25.01°, 25.79°, 26.70°, 28.65°, and 30.56° in an X-ray powder diffraction pattern obtained by using Cu-Kα radiation.

**Table 14. XRPD Data on Type XIII Crystal**

| Peak Position [°2θ] | Relative Peak Intensity [%] |
|---|---|
| 6.15 | 6.08 |
| 7.81 | 6.94 |
| 9.46 | 100.00 |
| 13.92 | 13.20 |
| 14.39 | 17.75 |
| 14.72 | 21.73 |
| 15.56 | 6.59 |
| 17.13 | 21.65 |
| 17.71 | 6.16 |
| 18.99 | 44.51 |
| 23.59 | 44.06 |
| 25.01 | 23.40 |
| 25.79 | 14.48 |
| 26.70 | 15.76 |
| 28.65 | 31.80 |
| 30.56 | 5.10 |

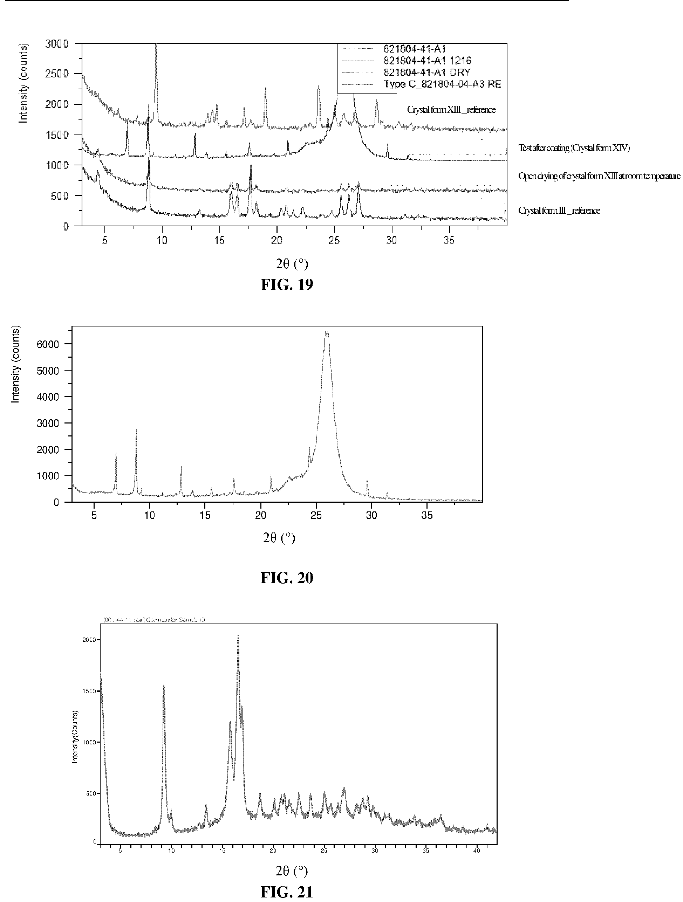

The type XIII crystal was unstable, and the crystal form was transformed after open dried at room temperature for about 2 h. Meanwhile, the type XIII crystal was coated to test XRPD, and new type XIV crystal could be observed. The type XIV crystal was presumed to be in an unstable intermediate state, and transformed into the type XIII crystal after removed from the solvent. The XRPD comparison diagram was as shown in FIG. 19. Rapid transformation of the crystal form of the type XIII crystal was observed at room temperature.

### Experiment 16

### Type XIV Crystal

The type XIII crystal was left in a solvent environment at room temperature and transformed into the type XIV crystal. The XRPD pattern was as shown in FIG. 20. The type XIV crystal was transformed into the type XIII crystal after removed from the solvent environment.

The type XIV crystal had characteristic peaks at diffraction angles of 2θ values = 6.94°, 8.75°, 9.21°, 11.16°, 12.82°, 13.82°15.56°, 17.59°, 19.67°, 20.93°, 22.51°, 24.35°, 29.58°, and 31.37° in an X-ray powder diffraction pattern obtained by using Cu-Kα radiation.

**Table 15. XRPD Data on Type XIV Crystal**

| Peak Position [°2θ] | Relative Peak Intensity [%] |
|---|---|
| 6.94 | 76.15 |
| 8.75 | 100.00 |
| 9.21 | 10.76 |
| 11.16 | 6.46 |
| 12.82 | 49.49 |
| 13.82 | 9.00 |
| 15.56 | 14.81 |
| 17.59 | 27.13 |
| 19.67 | 4.02 |
| 20.93 | 36.18 |
| 22.51 | 25.25 |
| 24.35 | 62.81 |
| 29.58 | 36.07 |
| 31.37 | 11.21 |

### Experiment 17

### Type XV Crystal

The type XII crystal was left at 40°C to 90°C for 3 to 12 days to obtain the type XV crystal. The XRPD pattern was as shown in FIG. 21.

The type XV crystal had characteristic peaks at diffraction angles of 20 values = 9.247°, 9.982°, 13.4°, 15.781°, 16.55°, 16.929°, 18.704°, 20.14°, 20.78°, 21.1°, 22.497°, 23.654°, 25.013°, 25.667°, 27.026°, 28.186°, 22.715°, 28.822°, 29.265°, and 29.762° in an X-ray powder diffraction pattern obtained by using Cu-Kα radiation.

**Table 16. XRPD Data on Type XV Crystal**

| Peak Position [°2θ] | Relative Peak Intensity [%] |
|---|---|
| 9.247 | 81.6 |
| 9.982 | 8 |
| 13.4 | 12.1 |
| 15.791 | 53.7 |
| 16.55 | 100 |
| 16.929 | 62 |
| 18.704 | 13.2 |
| 20.14 | 8.2 |
| 20.78 | 9.9 |
| 21.1 | 10.5 |
| 21.54 | 7.8 |
| 22.497 | 12.8 |
| 23.654 | 12.6 |
| 25.013 | 13.2 |
| 25.667 | 6.3 |
| 27.026 | 14.9 |
| 28.186 | 5.4 |
| 28.822 | 9.9 |
| 29.265 | 10.2 |
| 29.762 | 6.1 |

The weight loss of the type XV crystal was 2.2% when heated to 120°C, and the crystal form was transformed.

## Claims

1. A type XVIII crystal of a compound (3), 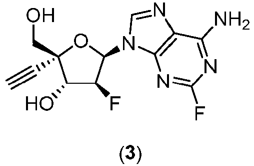 the type XVIII crystal having characteristic peaks at diffraction angles of 2θ values = 9.325°, 15.787°, and 16.55° in an X-ray powder diffraction pattern obtained by using Cu-Kα radiation, wherein the 20 values are allowed to vary within an error range.

2. The type XVIII crystal of the compound (3) according to claim 1, having diffraction peaks shown in the following table:
| Pos. [°2θ] | Rel. Int. [%] |
|---|---|
| 9.325 | 27.9 10 |
| 15.787 | 14.4 |
| 16.55 | 31.6 |

3. A method for preparing the type XVIII crystal of the compound (3) according to claim 1 or 2, comprising: leaving a type XVI or XVII crystal at 40°C to 160°C, preferably 60°C to 90°C, more preferably 60°C for 1 to 10 days, preferably 5 to 10 days, more preferably 1 day to obtain the type XVIII crystal.

4. The method according to claim 3, wherein the preparation step of the type XVI crystal comprises: suspending and stirring the compound (3) in an ethanol/water mixed solvent at a volume ratio of 10:1-90, preferably 10:1-50, more preferably 10:1-20 for 1-72 h, preferably 1-50 h, more preferably 10-40 h, more preferably 24 h, and filtering to obtain the type XVI crystal.

5. The method according to claim 3, wherein the type XVII crystal is obtained by heating the type XVI crystal to 40°C to 58°C, preferably 55°C in a nitrogen atmosphere and holding for appropriate time; or obtained by vacuum drying the type XVI crystal at room temperature for 5-72 h, preferably 10-50 h, more preferably 24 h.

6. A pharmaceutical composition, comprising the type XVIII crystal of the compound (3) according to claim 1 or 2, and an optional pharmaceutical excipient.

7. Use of the type XVIII crystal of the compound (3) according to claim 1 or 2 or the pharmaceutical composition according to claim 6 in the preparation of a pharmaceutical or pharmaceutical formulation for resisting HIV or treating AIDS.
